# DEMANDE DE BREVET EUROPEEN

(11) **EP 1 607 084 A1**
(43) Date de publication de la demande: **21.12.2005**
(21) Numéro de dépôt: 05291148.4
(22) Date de dépôt: 27.05.2005
(51) Int. Cl.: A61K 7/13, A61K 7/135, A61K 7/09

(54) **Composition pour le traitement de matières kératiniques comprenant un composé polycarboxylique particuier et un tensioactif mono- ou poly- glycérole et procédés la mettant en oeuvre**

(30) Priorité: 28.05.2004 FR 0405831
(71) Demandeur: L'OREAL, 75008 Paris (FR)
(72) Inventeur: Legrand, Frédéric, 92400 Courbevoie (FR); Cottard, François, 92400 Courbevoie (FR)
(74) Mandataire: Wattremez, Catherine

(57) **Abrégé**

La présente invention a pour objet une composition destinée au traitement des matières kératiniques humaines, comprenant un composé choisi parmi l'acide éthylènediamine-N,N'-disuccinique, un de ses sels, ou leur mélange, ledit composé étant associé à au moins un tensioactif mono- ou poly-glycérolé.

Elle concerne de plus un procédé de traitement des matières kératiniques, sèches ou humides, dans lequel on applique la composition selon l'invention.

Elle concerne aussi des procédés de déformation, décoloration, coloration de fibres kératiniques humaines la mettant en oeuvre.

## Description

La présente invention a pour objet une composition destinée au traitement des matières kératiniques humaines, comprenant un composé portant quatre fonctions acide carboxylique, sous forme acide ou sous forme de sel, ledit composé étant associé à au moins un tensioactif mono- ou poly-glycérolé. Elle concerne de plus des procédés de traitement des matières kératiniques consistant à appliquer ladite composition. Elle a par ailleurs pour objet des procédés de déformation, décoloration, coloration de fibres kératiniques humaines mettant en oeuvre la composition selon l'invention.

Depuis longtemps, on a cherché à modifier l'aspect, la couleur des fibres kératiniques humaines, et notamment des cheveux.

Ainsi, on peut mettre en oeuvre des procédés de déformation permanente réalisés habituellement en deux temps.

Plus spécialement, on effectue une première étape, durant laquelle les ponts disulfures présents dans les fibres sont ouverts, habituellement en présence d'un agent réducteur.

Avant, après, ou simultanément à la réduction de ces ponts disulfures, la fibre est mise en forme de la manière souhaitée (bigoudi, lissage).

Une fois cette première étape effectuée, il est nécessaire de mettre en oeuvre une étape durant laquelle les ponts disulfures sont reformés afin de stabiliser la forme obtenue. Cette étape de fixation de la forme est mise en oeuvre au moyen d'une composition comprenant un agent oxydant.

En ce qui concerne les procédés de décoloration des fibres, ces traitements sont effectués par oxydation du pigment "mélanine" aboutissant à la solubilisation et l'élimination partielle ou totale de ce pigment dans la fibre.

Dans ce but, on utilise des compositions décolorantes contenant au moins un réactif peroxygéné et au moins un agent alcalin comme activateur de ces composés peroxygénés. Ces compositions sont par la suite associées au moment de l'emploi à une composition aqueuse de peroxyde d'hydrogène.

Selon une autre possibilité, le but recherché par les procédés de décoloration peut être de décaper la fibre, en d'autre termes de la débarrasser des colorants artificiels présents.

Ce type de décoloration peut être fait soit en présence d'un agent oxydant, comme précédemment résumé, ou bien en présence d'un agent réducteur. L'agent actif mis en oeuvre est en fait choisi de manière classique par l'homme du métier, en fonction de la nature du colorant à éliminer.

Enfin, dans le domaine de la coloration, on connaît plusieurs types de procédés parmi lesquels figurent la coloration permanente (ou coloration d'oxydation) et la coloration directe (ou coloration semi-permanente) avec ou sans effet d'éclaircissement des fibres.

Dans le domaine de la coloration d'oxydation, les composés utilisés sont des précurseurs de colorants d'oxydation, plus particulièrement des bases d'oxydation éventuellement associées à un ou plusieurs coupleurs. Ces composés sont des substances incolores ou peu colorées qui, par un processus de condensation oxydative, en présence d'un agent oxydant, conduisent à des composés qui colorent les fibres.

Dans le domaine de la coloration semi-permanente, la composition appliquée sur les fibres comprend au moins des colorants directs, qui sont des composés colorants et colorés, et peut éventuellement aussi comprendre au moins un agent oxydant, si l'on souhaite obtenir un effet éclaircissant associé à la coloration.

Quel que soit le résultat souhaité, les compositions employées doivent satisfaire à des critères relativement contraignants du fait qu'elles sont appliquées directement sur les fibres et entrent en contact avec la peau, et qu'elles contiennent des ingrédients ou sont utilisées dans des conditions telles (notamment de pH) qui peuvent entraîner des réactions de sensibilisation de la peau, ainsi qu'une dégradation à la longue, des fibres traitées.

Des progrès importants ont été réalisés dans ces divers domaines de traitements des fibres kératiniques. Ainsi, les compositions contiennent de nombreux additifs qui, par exemple, visent à régler la rhéologie de la composition afin qu'elle puisse être appliquée facilement et rapidement, sans couler hors des zones à traiter pendant le temps de pose. Il existe aussi d'autres additifs qui ont pour objectif de limiter ou corriger des effets de dégradation de la fibre kératinique occasionnés par les procédés.

Cependant, dans certains cas, les additifs présents ne sont pas sans effet sur les résultats mêmes du traitement.

C'est pourquoi on cherche toujours à améliorer la puissance, la sélectivité, la ténacité des colorations obtenues, l'homogénéité des décolorations, la tenue des formes données aux fibres.

Ainsi, la présente invention a pour objet de proposer des compositions qui résolvent les problèmes ci-dessus.

Un premier objet de l'invention est donc constitué par une composition destinée au traitement de matières kératiniques humaines, comprenant :
- au moins un composé choisi parmi l'acide éthylènediamine-N,N'-disuccinique, un de ses sels, ou leur mélange,
- au moins un tensioactif mono- ou poly- glycérolé.

Un autre objet de la présente invention consiste à appliquer la composition selon l'invention sur des matières kératiniques, plus particulièrement humaines, et notamment sur des fibres.

Elle a de même pour objet des procédés de déformation permanente des fibres, de décoloration, de coloration, mettant en oeuvre la composition selon l'invention.

Mais d'autres caractéristiques et avantages de la présente invention apparaîtront plus clairement à la lecture de la description et des exemples qui vont suivre.

Dans ce qui va suivre et à moins que le contraire ne soit précisé, les bornes délimitant un domaine de valeurs sont comprises dans ce domaine.

Par ailleurs, par matières kératiniques humaines, on désigne la peau et les fibres kératiniques, et encore plus particulièrement des cheveux.

Dans le cas de compositions prêtes à l'emploi, dans le cas où celles-ci peuvent être stockées telles quelles, les teneurs détaillées pour les compositions selon l'invention restent valables, à l'exception du fait qu'elles sont exprimées par rapport à la composition prête à l'emploi.

Dans le cas de compositions prêtes à l'emploi obtenues par mélange extemporané de plusieurs compositions, ces teneurs sont à modifier en fonction du taux de dilution pour obtenir ladite composition prête à l'emploi.

Ainsi que cela a été indiqué auparavant, la composition selon l'invention comprend au moins un composé choisi parmi l'acide éthylènediamine-N,N'-disuccinique, un de ses sels, ou leur mélange.

Les sels sont de préférence des sels de métaux alcalins (comme par exemple le sodium, le potassium), de métaux alcalino-terreux (notamment le calcium, le magnésium), d'ammonium substitués ou non par un à trois radicaux alkyle, identiques ou différents, comprenant 1 à 6 atomes de carbone, de préférence de 1 à 4 atomes de carbone, éventuellement porteurs d'au moins un radical hydroxyle.

De préférence, le composé entrant dans la composition selon l'invention se présente sous la forme d'un sel de sodium.

Conformément à un mode de réalisation particulier de l'invention, la teneur en acide éthylènediamine-N,N'-disuccinique, sous forme acide ou sel, ou en mélange, représente entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

La composition selon l'invention comprend en outre, au moins un tensioactif mono- ou poly- glycérolé. Ledit tensioactif est avantageusement non ionique.

Le tensioactif entrant dans la composition selon l'invention est plus particulièrement choisi parmi les alcools gras mono- ou poly- glycérolés. Ces derniers correspondent plus spécialement à la formule (I) suivante : 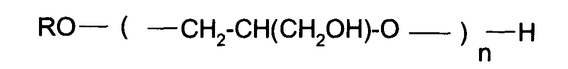 dans laquelle :
R représente un radical saturé ou insaturé, linéaire ou ramifié, comportant de 8 à 40 atomes de carbone et de préférence de 10 à 30 atomes de carbone ;
n représente un nombre compris entre 1 et 30 et de préférence entre 1 et 10.

A titre d'exemples plus précis de ce type de composés, on peut citer entre autres, sans intention de s'y limiter, l'alcool laurique à 4 moles de glycérol (nom INCl: polyglyceryl-4 lauryl ether), l'alcool oléique à 4 moles de glycérol (nom INCI : polyglyceryl-4 oleyl ether), l'alcool oléique à 2 moles de glycérol (Nom INCI : polyglyceryl-2 oleyl ether), l'alcool cétéarylique à 2 moles de glycérol, l'alcool cétéarylique à 6 moles de glycérol , l'alcool oléocétylique à 6 moles de glycérol, et l'octadécanol à 6 moles de glycérol.

Il est à noter que l'alcool gras peut représenter un mélange d'alcools gras au même titre que la valeur de n représente une valeur statistique, ce qui signifie que dans un produit commercial peuvent coexister plusieurs espèces d'alcools gras polyglycérolés sous forme d'un mélange.

Avantageusement, les alcools gras mono- ou poly- glycérolés représentent de 0,01 à 30% en poids par rapport au poids de la composition, plus particulièrement de 0,05 à 20% en poids par rapport au poids de la composition, et de préférence de 0,1 à 15% en poids par rapport au poids de la composition.

La composition selon l'invention peut en outre comprendre au moins un agent oxydant.

Habituellement, l'agent oxydant est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges.

La teneur en agent oxydant est en général comprise entre 1 et 60 % en poids, de préférence entre 3 et 50 % en poids par rapport au poids de la composition.

La composition comprenant l'agent oxydant peut se trouver sous la forme d'une solution, d'une émulsion, ou encore d'une pâte.

Dans ce dernier cas, la pâte ne comprend pas d'eau ou en comprend une teneur inférieure ou égale à 1 % en poids par rapport au poids de la composition, de préférence inférieure ou égale à 0,5 % en poids par rapport au poids de la composition.

Au cas où la composition oxydante est destinée à être utilisée pour la décoloration ou le décapage de la fibre kératinique, elle comprend alors de préférence au moins un persel, au moins un agent alcalin et de 15 à 35 % d'au moins un liquide inerte organique.

Usuellement, l'agent alcalin est choisi parmi l'urée ; les sels d'ammonium, comme les chlorures, les sulfates, les phosphates, les nitrates ; les silicates, les phosphates, les carbonates de métaux alcalins (tels que le sodium, le potassium) ou alcalino-terreux (comme notamment le magnésium) seuls ou combinés.

Il est en général présent à hauteur de 0,01 à 40 % en poids, de préférence de 0,1 à 30 % en poids, par rapport au poids de la composition.

Quant au liquide inerte organique, ce dernier est un composé ou un mélange de composés liquides à température ambiante (entre 15 et 25°C) et à pression atmosphérique. Avantageusement, il est choisi parmi les polydécènes, les mono- et polyesters d'acides carboxyliques, les mono- ou poly- esters de sucres d'acides en C₈-C₃₀, les éthers cycliques, les esters cycliques, les huiles silicones, les huiles minérales, les huiles végétales, ou leurs mélanges.

Notons que des compositions sous forme de pâte sont obtenues par tout moyen classique, par exemple par dispersion des composés pulvérulents dans le liquide inerte, dans lequel on a préalablement dispersé ou mélangé les autres composés liquides de la composition de décoloration, ou encore par extrusion.

La composition selon l'invention peut comprendre par ailleurs au moins un agent réducteur, que ce soit pour le décapage ou pour la déformation permanente des fibres.

Plus particulièrement, l'agent réducteur est choisi parmi les réductones ou les composés comprenant du soufre, et notamment des composés présentant au moins une fonction thiol, (di)sulfure, sulfite, sulfinique, sous forme de sel ou non.

Parmi les réductones, on peut citer entre autres l'acide (iso)ascorbique, l'acide érythorbique, sous forme acide, estérifiée ou encore salifiée. De manière particulièrement avantageuse conviennent l'acide ascorbique, l'acide isoascorbique, sous forme acide ou de l'un de ses sels comme le sel de sodium.

Parmi les thiols utilisables en tant que composé réducteur, on peut citer l'acide thioglycolique, le β-mercaptoéthanol, l'acide thiolactique, leurs sels de métal alcalin ou alcalino-terreux (comme le sodium, le potassium, le calcium) et leurs esters ; la cystéine, la cystéamine et leurs dérivés ; l'homocystéine et l'un de ses sels ; le mercaptoaldéhyde ; la pénicillamine ; la glutathione ; le thioglycolate de glycérol. Ces composés pouvant être utilisés seuls ou en mélanges.

En ce qui concerne les sulfites utilisables en tant que réducteurs, parmi lesquels figurent aussi les bisulfites, les hydrosulfites, conviennent les sels de métaux alcalins, alcalino-terreux ou d'ammonium, ainsi que leurs mélanges. Plus particulièrement, on peut citer le sulfite de sodium et l'hydrosulfite de sodium.

En ce qui concerne les sulfures, parmi lesquels figurent aussi les disulfures, on peut citer notamment la cystine.

Pour ce qui a trait aux composés sulfiniques, on peut citer les composés correspondants à la formule suivante : 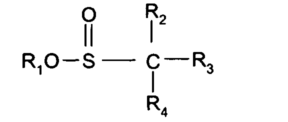 dans laquelle,
R₁ est choisi parmi, un atome d'hydrogène, un ion NH₃⁺, un ion de métal monovalent ou un équivalent ionique de métal bivalent des groupes la, IIa, IIb, IVa et VIIIb du système périodique des éléments,
R₂ est choisi parmi, un radical OH, un radical NR₅R₆ dans lequel R₅ et R₆, identiques ou différents, sont choisis parmi un atome d'hydrogène ou un radical alkyle en C₁-C₆,
R₃ est choisi parmi, un atome d'hydrogène, un radical alkyle ou alcényle ou cycloalkyle en C₁-C₆ ; aryle non substitué ou substitué par 1 à 3 substituants choisis, identiques ou différents, parmi les radicaux OH, alkyle en C₁-C₆, O-alkyle en C₁-C₆, halogène ou CF₃,
R₄ est choisi parmi, un radical COOR₁, SO₃R₁, COR₅, CONR₅R₆ ou COOR₅ ou dans lesquels R₁, R₅, et R₆ ont les significations précédentes,
et R₄ désigne également un atome d'hydrogène, lorsque R₃ désigne un radical aryle, et en particulier, un radical aryle substitué comme décrit précédemment,
et les sels desdits composés cosmétiquement acceptables.

De préférence, les sels des composés sulfiniques peuvent être choisis parmi les sulfinates alcalins (Na, K), alcalinoterreux (Ca, Mg) ou de zinc.

Les dérivés d'acides sulfiniques de formule ci-dessus selon l'invention sont des composés connus, décrits et préparés dans la demande de brevet WO 99/18067 et WO02/30369.

De manière avantageuse, on peut mettre en oeuvre des dérivés d'acide sulfinique de formule précitée pour laquelle R₁ est choisi parmi un ion NH₃⁺, un ion de métal alcalin, un équivalent ionique de métal alcalino-terreux ou de zinc, R₂ est un radical OH ou un radical NH₂, R₃ est choisi parmi, un atome d'hydrogène, un radical alkyle non substitué ou substitué par un ou deux radicaux OH ou un ou deux radicaux alkyle en C₁-C₆ ou alcoxy en C₁-C₆, R₄ est un radical COOR₁ ou COOR₅, dans lesquels R₁ et R₅ ont les mêmes significations que celles indiquée plus haut.

Conformément à un mode de réalisation plus particulier, le dérivé d'acide sulfinique est tel que R₁ représente le sodium, R₂ représente OH, R₃ représente un atome d'hydrogène et R₄ représente COONa. Sont aussi susceptibles de convenir, des mélanges comprenant 40 à 60% dudit composé, 10 à 20% de NaSO₃-CHOH-COONa et de 30 à 40% de Na₂SO₃ (par exemple le produit Brüggolite® FF6 de la société Bruggemann : 50% du composé NaSO₂-CHOH-COONa ; 15% du composé NaSO₃-CHOH-COONa et 35% de Na₂SO₃).

On pourra aussi mettre en oeuvre à titre d'agent réducteur, les dérivés d'acide sulfinique notamment décrits dans WO03/026597 et WO03/041668.

De préférence, l'agent réducteur est choisi parmi l'acide ascorbique, l'hydroxyméthane sulfinate de sodium, les thiols, le sulfite de sodium, seuls ou en mélanges.

La teneur en agent réducteur dans la composition est avantageusement comprise entre 0,01 et 10 % en poids par rapport au poids de la composition, de préférence entre 0,1 et 5 % en poids par rapport au poids de la composition.

Notons que la composition peut se trouver sous la forme d'une lotion épaissie, d'une crème, d'un gel, ou de toute autre forme appropriée pour l'application ultérieure de cette composition.

Ainsi, lorsque la composition est destinée à une opération de défrisage ou de décrêpage de fibres, elle se trouve de préférence sous forme d'une crème épaissie de façon à maintenir les fibres à traiter aussi raides que possible pendant le traitement. On réalise ces crèmes sous forme d'émulsions "lourdes", obtenues par exemple en émulsionant une phase aqueuse, comprenant notamment l'agent réducteur, et une phase huileuse (huile végétale, huile de paraffine, esters d'acides gras, cire, par exemple).

On peut également utiliser des liquides ou des gels contenant des agents épaississants tels que des polymères ou des copolymères carboxyvinyliques qui "collent" les fibres et les maintiennent dans la position lisse pendant le temps de pose.

Il est à noter que la composition peut de même comprendre au moins un colorant direct ou au moins une base d'oxydation éventuellement associée à au moins un coupleur, ou leurs mélanges.

En ce qui concerne les colorants directs, on peut utiliser des espèces anioniques, cationiques ou non ioniques.

Avantageusement, ils peuvent être choisis parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

S'ils sont présents, la teneur en colorant(s) direct(s) dans la composition varie en général de 0,001 à 20% en poids par rapport à la composition, et de préférence de 0,01 à 10% du poids total de la composition.

En ce qui concerne les bases d'oxydation, elles peuvent être choisies notamment ortho-phénylènediamines, les para-phénylènediamines, les bis-phénylalkylènediamines, les para-aminophénols, les bis-para-aminophénols, les ortho-aminophénols, les bases hétérocycliques et leurs sels d'addition avec un acide, ainsi que leurs mélanges.

En général, lorsqu'elles sont présentes dans la composition, la teneur en base(s) d'oxydation représente de 0,0005 à 12% en poids par rapport au poids de la composition, avantageusement de 0,005 à 8% en poids par rapport au poids de la composition.

Quant aux coupleurs éventuellement associées aux bases d'oxydation précitées, on peut mettre en oeuvre un ou plusieurs composés choisis parmi les m-aminophénols, les m-phénylènediamines, les m-diphénols, les naphtols, les coupleurs hétérocycliques, leurs sels d'addition avec un acide, ainsi que leurs mélanges.

Lorsqu'ils sont présents, ces coupleurs représentent plus spécialement de 0,0001 à 10% en poids par rapport au poids de la composition, et de préférence de 0,005 à 5% en poids par rapport au poids de la composition.

D'une manière générale, les sels d'addition avec un acide des bases d'oxydation et coupleurs sont notamment choisis parmi les chlorhydrates, les bromhydrates, les sulfates, les citrates, les succinates, les tartrates, les lactates, les tosylates, les benzènesulfonates, les phosphates et les acétates.

En outre, la composition selon l'invention peut de plus comprendre au moins un tensioactif, de préférence non ionique, anionique, amphotère ou zwittérionique.

Parmi les tensioactifs non ioniques, on peut citer les alcools, les alpha-diols, les alkylphénols polyéthoxylés et/ou polypropoxylés, ayant une chaîne hydrocarbonée comprenant par exemple de 8 à 30 atomes de carbone, de préférence de 8 à 18 atomes de carbone, le nombre de groupements oxyde d'éthylène et/ou oxyde de propylène pouvant aller notamment de 2 à 50.

On peut également citer les copolymères d'oxyde d'éthylène et de propylène, les condensats d'oxyde d'éthylène et/ou de propylène sur des alcools gras ; les amides gras polyéthoxylés ayant de préférence de 2 à 30 moles d'oxyde d'éthylène ; les esters d'acides gras de sorbitan oxyéthylénés ayant de 2 à 30 moles d'oxyde d'éthylène ; les esters d'acides gras de sucrose, les esters d'acides gras et de polyéthylèneglycol, les alkylpolyglycosides, les dérivés de N-alkyl glucamine, etc.

Parmi les tensioactifs anioniques, on peut citer entre autres les sels (en particulier les sels alcalins, notamment de sodium, de magnésium, les sels d'ammonium, les sels d'amines, les sels d'aminoalcools) d'alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄) sulfosuccinates, alkyl(C₆-C₂₄) éthersulfosuccinates, alkyl(C₆-C₂₄) amidesulfosuccinates ; alkyl(C₆-C₂₄) sulfoacétates ; acyl(C₆-C₂₄) sarcosinates et glutamates ; esters d'alkyl(C₆-C₂₄)polyglycosides carboxyliques ; alkylsulfosuccinamates ; acyliséthionates et N-acyltaurates ; le radical alkyle ou acyle de tous ces différents composés comportant de préférence de 12 à 20 atomes de carbone, et le radical aryle désignant de préférence un groupement phényle ou benzyle.

Conviennent aussi les sels d'acides gras tels que les sels des acides oléique, ricinoléique, palmitique, stéarique, les acides d'huile de coprah hydrogénée ou non ; les acides d'alkyl D-galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

En ce qui concerne les tensioactifs amphotères ou zwittérioniques conviennent notamment les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer amphocarboxyglycinates et amphocarboxypropionates, comme par exemple les disodium cocoamphodiacetate, disodium lauroamphodiacetate, disodium caprylamphodiacetate, disodium cocoamphodipropionate, disodium lauroamphodipropionate, etc. (produits de la gamme Miranol®, notamment Miranol® C2M concentré commercialisés la société Rhodia Chimie).

La teneur en tensioactif dans la composition représente habituellement de 0,01 à 40 % en poids et de préférence de 0,5 à 30 % en poids, du poids total de la composition.

La composition conforme à l'invention peut renfermer au moins un agent épaississant, associatif ou non. Ainsi, les compositions peuvent comprendre notamment à titre de polymères épaississants des composés choisis parmi les dérivés de cellulose, comme par exemple les hydroxypropyl ou carboxyméthyl celluloses ; les dérivés de guar ; les gommes d'origine microbienne, comme les gommes de scléroglucane, de xanthane ; les gommes issues d'exudats végétaux telles que les gommes arabiques, Ghatti, Karaya, Tragacanthe ; ainsi que les épaississants synthétiques comme les homopolymères d'acide acrylique, d'acrylate, d'acrylamide, d'acide 2-acrylamido-2-méthyl-propane sulfonique, réticulés ou non ; les pectines ; les alginates.

La composition peut aussi comprendre un ou plusieurs polymères associatifs en tant qu'épaississant comme notamment, les dérivés de polyuréthanes associatifs, les dérivés de celluloses associatifs, les dérivés de polyvinyllactames associatifs et les dérivés de polyacides insaturés associatifs, et leurs mélanges.

Habituellement, au cas où il est présent, la teneur en polymère épaississant, associatif ou non, représente de 0,01 et 10% en poids par rapport au poids de la composition, et en particulier entre 0,1 et 5% en poids par rapport au poids de la composition.

La composition conforme à l'invention peut également comprendre divers adjuvants utilisés classiquement, tels que des polymères substantifs cationiques ou amphotères, des polymères anioniques, cationiques, non ioniques, amphotères, zwittérioniques, des agents épaississants minéraux, des agents antioxydants, des acides alpha-oxocarboxyliques (par exemple l'acide oxalique, l'acide glyoxalique, l'acide pyruvique ou l'acide alpha-cétoglutarique), des agents de pénétration, des parfums, des tampons, des acides aminés basiques comme l'arginine notamment, des agents dispersants, des agents peptisants, des agents de conditionnement tels que par exemple des cations, des silicones volatiles ou non volatiles, modifiées ou non modifiées, des agents filmogènes, des céramides, des vitamines ou provitamines, des agents conservateurs, des agents stabilisants, des agents opacifiants ou matifiants comme le dioxyde de titane, des charges minérales, comme les argiles, les silices notamment pyrogénées à caractère hydrophile ou hydrophobe, des polymères liants tels que la vinylpyrrolidone, des lubrifiants comme les stéarates, des agents de contrôle du dégagement d'oxygène tels que le carbonate ou l'oxyde de magnésium, les filtres solaires, etc.

Les adjuvants cités ci-dessus sont en général présents en quantité comprise pour chacun d'eux entre 0,01 et 20% en poids par rapport au poids de la composition.

Le milieu de la composition est un milieu cosmétiquement acceptable.

Selon une première possibilité, il est constitué par de l'eau ou par un mélange d'eau et d'un ou plusieurs solvants organiques usuels.

Plus particulièrement, les solvants organiques sont choisis parmi les monoalcools ou les diols différents des polyols de formule (I), linéaire ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition.

Selon une deuxième possibilité, le milieu de la composition selon l'invention ne comprend pas d'eau ou comprend une teneur en eau inférieure ou égale à 1 % en poids par rapport au poids de la composition, de préférence inférieure ou égale à 0,5 % en poids par rapport au poids de la composition.

Dans ce cas, le milieu de la composition est un liquide organique inerte choisi parmi ceux mentionnés auparavant.

La teneur en ce type de composés est comprise entre 15 et 35 % en poids par rapport au poids de la composition.

Le pH de la composition selon l'invention peut être déterminé de manière classique par l'homme du métier et dépend entre autres, du domaine de stabilité du ou des agents réducteurs présents dans la composition. A titre indicatif, le pH de la composition selon l'invention est généralement compris entre 3 et 14 environ, et de préférence entre 5 et 12 environ.

Il peut être ajusté à la valeur désirée au moyen d'agents acidifiants ou alcalinisants.

Parmi les agents acidifiants, on peut citer, à titre d'exemples, les acides minéraux ou organiques comme l'acide chlorhydrique, l'acide orthophosphorique, l'acide sulfurique, l'acide acétique.

Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 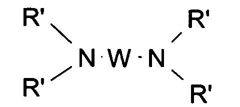 dans laquelle W est un reste propylène éventuellement substitué par un groupement hydroxyle ou un radical alkyle en C₁-C₆ ; les radicaux R', identiques ou différents, représentent un atome d'hydrogène, un radical alkyle en C₁-C₆ ou hydroxyalkyle en C₁-C₆.

La composition selon l'invention peut se trouver sous la forme de produits de lavage, comme des shampooings, mais aussi des après-shampooings, des produits de mise en forme (comme par exemple des laques, des mousse, etc.), des produits de coloration, de décoloration, de mise en forme des fibres kératiniques.

La présente invention a de plus pour objet des procédés mettant en oeuvre la composition selon l'invention.

Ainsi, le procédé consiste à mettre en contact les matières kératiniques, sèches ou humides, avec la composition selon l'invention qui vient d'être décrite.

Généralement, le temps de pose de la composition est compris entre quelques secondes et 30 minutes, de préférence entre 3 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

De préférence, les matières kératiniques sont rincées, avantageusement à l'eau, et si nécessaire, lavées avec un shampooing puis rincées à nouveau à l'eau

Selon un premier mode de réalisation particulier, l'invention a pour objet un procédé de déformation permanente des fibres kératiniques humaines consistant à mettre en contact dans une première étape, une composition réductrice avec lesdites fibres kératiniques, ces dernières étant mises en forme avant, pendant ou après l'application de la composition réductrice ; puis dans une deuxième étape, à appliquer une composition oxydante sur les fibres ainsi traitées ; le composé portant les quatre fonctions carboxyliques et le tensioactif (poly)glycérolé se trouvant dans la composition réductrice et/ou dans la composition oxydante.

La première étape consiste donc à appliquer sur lesdites fibres, sèches ou humides, une composition réductrice. Cette composition peut éventuellement comprendre le composé portant les quatre fonctions carboxyliques et le tensioactif mono- ou poly-glycérolé.

De préférence, selon cette première variante, le pH de la composition réductrice appliquée est d'au moins 7.

Si l'opération est destinée à friser les fibres, ces dernières sont mises sous tension au moyen de bigoudis, avant, pendant ou après l'application de la composition réductrice. En cas de défrisage, la composition est appliquée sur les fibres en les lissant.

Le temps de pose est en général compris entre quelques secondes et 30 minutes, de préférence entre 3 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

Il peut être approprié d'effectuer une étape intermédiaire, entre l'étape de réduction et celle de fixation (d'oxydation), d'appliquer de la chaleur.

Une fois le temps de pose écoulé et éventuellement cette étape intermédiaire réalisée, on applique sur les fibres une composition oxydante.

Comme indiqué auparavant, la composition oxydante peut éventuellement comprendre le composé portant les quatre fonctions carboxyliques et le tensioactif (poly)glycérolé.

De manière classique, la composition comprend, à titre d'agent oxydant, du peroxyde d'hydrogène, un bromate alcalin, un persel ou un polythionate.

Le pH de cette composition oxydante est généralement compris entre 2 et 10.

De préférence, on utilise du peroxyde d'hydrogène avec un pH avantageusement compris entre 2 et 7.

La composition oxydante peut de plus être obtenue en mélangeant au moins deux compositions, l'une d'elles comprenant l'agent oxydant, l'autre comprenant au moins un agent alcalin.

La composition oxydante peut se trouver sous avantageusement sus la forme d'une émulsion, habituellement épaissie.

Le temps de pose de la composition oxydante est en général compris entre 3 et 30 minutes, de préférence entre 5 et 15 minutes.

Le plus souvent, les fibres imprégnées de la composition oxydante sont rincés, généralement à l'eau. On sépare, avant ou après le rinçage, les fibres kératiniques des moyens nécessaires à leur mise sous tension.

Les fibres sont ensuite éventuellement lavées au shampooing, rincées puis séchées ou laissées à sécher.

Ce qui a été indiqué plus haut quant à la nature des divers ingrédients entrant dans les compositions, ainsi que leurs quantités respectives, reste valable et ne sera pas repris dans cette partie de la description.

Conformément à une autre variante, le procédé de mise en forme de fibres kératiniques humaines consiste à appliquer sur les fibres kératiniques, sèches ou humides, une composition comprenant, en plus du ou des composés et tensioactifs (poly)glycérolés précités, au moins un agent acalinisant, tel que de préférence l'hydroxyde de sodium.

Ce type de composition est particulièrement approprié lorsque l'on souhaite faire une étape de défrisage des cheveux.

Habituellement, les fibres sont lissées pendant ou après l'application de la composition.

Le temps de pose est en général compris entre 3 et 30 minutes, de préférence entre 5 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

Les fibres imprégnées de la composition sont rincés soigneusement, généralement à l'eau, de préférence lavées au shampooing, rincées puis séchées ou laissées à sécher.

Selon un deuxième mode de réalisation particulier, l'invention a pour objet un procédé de décoloration des fibres kératiniques consistant à mettre en contact la composition prête à l'emploi, avec lesdites fibres pendant une durée suffisante pour obtenir l'effet souhaité.

Conformément à une première variante de ce deuxième mode de réalisation, la composition prête à l'emploi comprend, outre le ou les composés portant les quatre fonctions carboxylique et le ou les tensioactifs (poly)glycérolés, au moins un agent oxydant.

Une première possibilité est constituée par un procédé mis en oeuvre à un pH acide (soit inférieur à 7). De préférence, l'agent oxydant est le peroxyde d'hydrogène.

Selon cette première possibilité, la composition appliquée sur les fibres kératiniques comprend au moins un agent oxydant, le composé et le tensioactif (poly)glycérolé.

Une deuxième possibilité est constituée par un procédé mis en oeuvre à un pH basique (soit supérieur ou égal à 7).

Dans ce cas, la composition prête à l'emploi appliquée sur les fibres est de préférence obtenue par mélange extemporané, avant l'application sur les fibres, d'une première composition comprenant au moins un persel, avec une deuxième composition aqueuse comprenant un agent oxydant, et éventuellement une troisième composition comprenant au moins un agent alcalin ; le(s) tensioactif(s) (poly)glycérolé(s) et le composé comportant quatre fonctions carboxyliques sous forme acide ou salifiée, se trouvant, ensemble ou non, dans l'une et/ou l'autre des compositions précitées.

Classiquement, la première composition se présente sous une forme anhydre, et comprend au moins un persel, éventuellement au moins un agent alcalin, et éventuellement au moins un liquide inerte.

La deuxième composition, qui est une composition aqueuse, comprend de préférence à titre d'agent oxydant, le peroxyde d'hydrogène. Avantageusement, e pH de cette composition est inférieur ou égal à 7.

Comme indiqué auparavant, on peut éventuellement mettre en oeuvre une troisième composition, qui se présente aussi sous une forme aqueuse, comprenant au moins un agent alcalin.

Les listes données des divers ingrédients que l'on peut utiliser dans la composition selon l'invention restent valables et l'on pourra s'y reporter.

La composition prête à l'emploi est donc appliquée sur les fibres kératiniques, sèches ou humides, puis laissée pendant un temps de pose suffisant pour obtenir la décoloration recherchée. A l'issue de cette période, la composition est éliminée par un rinçage à l'eau, suivi d'un lavage avec un shampooing, puis éventuellement d'un séchage.

Habituellement, le temps de pose est compris entre quelques secondes et 30 minutes, de préférence entre 3 et 15 minutes.

La température à laquelle la composition est laissée agir est en général comprise entre la température ambiante (15 à 25°C) et 220°C, plus particulièrement entre la température ambiante et 80°C, de préférence entre 15 et 40 °C.

Conformément à une seconde variante de ce deuxième mode de réalisation, la composition prête à l'emploi comprend au moins un agent réducteur.

Cette variante est tout particulièrement appropriée lorsque l'on souhaite retirer une coloration artificielle, dans la mesure où celle-ci peut être éliminée en mettant en oeuvre une réaction de réduction. On parle alors de décapage.

Dans ce cas, la composition prête à l'emploi est appliquée sur des fibres teintes au moyen de colorants d'oxydation (base et/ou coupleur), de colorants directs, ou encore d'une combinaison de ces deux familles de colorants.

Dans le cas plus particulier où la composition prête à l'emploi comprend à titre d'agent réducteur, au moins une réductone ou dérivé d'acide sulfinique, le pH de la composition prête à l'emploi est de manière avantageuse inférieure à 7, de préférence compris entre 3 et 7. Le pH de la composition peut être adapté de manière classique en utilisant, selon les besoins, un ou plusieurs agents alcalinisants ou acidifiants, tels que notamment ceux listés plus haut.

Le procédé consiste donc à appliquer sur les fibres kératiniques la composition prête à l'emploi puis à laisser poser la composition pendant un temps suffisant pour obtenir la décoloration, à rincer les fibres pour en éliminer la composition, à les laver avec un shampooing, à les rincer et éventuellement les sécher.

Habituellement, le temps de pose varie entre quelques secondes et 60 minutes, de préférence entre 1 et 60 minutes, et plus préférentiellement entre 10 et 50 minutes.

La température à laquelle la composition est laissée agir est en général comprise entre la température ambiante (15 à 25°C) et 220°C, plus spécialement entre la température ambiante et 80°C, et de préférence entre 15 et 40 °C.

Selon un troisième mode de réalisation particulier, l'invention a pour objet un procédé de coloration des fibres kératiniques humaines dans lequel on met en contact la composition prête à l'emploi avec lesdites fibres pendant une durée suffisante pour obtenir l'effet souhaité.

Selon une première possibilité, la composition prête à l'emploi ne comprend pas d'agent oxydant, elle peut être appliquée directement sur les fibres.

Dans ce cas, la composition prête à l'emploi comprend, outre le(s) composé(s) portant quatre fonctions carboxyliques, le(s) tensioactif(s) (poly)glycérolé(s), au moins un colorant direct. Cette composition peut être stockée telle quelle ou bien résulter du mélange extemporané d'au moins une composition comprenant au moins un tensioactif (poly)glycérolé, d'au moins une composition comprenant au moins un colorant direct : le composé portant quatre fonctions carboxyliques pouvant se trouver dans l'une et/ou l'autre des compositions. De manière avantageuse, la composition prête à l'emploi peut être stockée telle quelle.

Dans le cas de cette première possibilité de procédé, on applique la composition prête à l'emploi pendant le temps nécessaire au développement de la coloration, puis à laisser sécher ou à sécher les fibres, sans rinçage final ou bien avec un rinçage.

On peut aussi envisager avec ou sans rinçage en fin de temps de pose, d'effectuer un lavage avec un shampooing des fibres, de les rincer et de les sécher ou les laisser sécher.

Le temps de pose est d'environ quelques secondes à 60 minutes, plus particulièrement de 5 à 60 minutes et de préférence 5 à 40 minutes.

La température nécessaire au développement de la coloration est généralement comprise entre la température ambiante (15 à 25°C) et 220°C, plus spécialement entre la température ambiante et 80°C, et avantageusement entre 15 et 40°C.

Selon une deuxième possibilité, la composition prête à l'emploi comprend au moins un agent oxydant. Comme indiqué auparavant, elle est de préférence obtenue par mélange extemporané, avant l'application, d'au moins une composition comprenant au moins un tensioactif (poly)glycérolé, au moins une base d'oxydation et/ou au moins un coupleur et/ou au moins un colorant direct avec au moins une composition oxydante ; le composé portant quatre fonctions acide carboxylique se trouvant dans l'une ou plusieurs des compositions précitées.

La composition oxydante employée dans ce cas est classique et correspond habituellement à une composition aqueuse comprenant un agent oxydant tel que listé auparavant.

La composition oxydante peut de même comprendre les additifs usuels dans le domaine, comme par exemple des agents tensioactifs, des liquides hydrophobes (si la composition est sous forme émulsionnée) ; des agents séquestrants ; des agents stabilisants le peroxyde d'hydrogène ; des agents anti-mousse, etc.

Comme précédemment, le temps de pose est d'environ quelques secondes à 60 minutes, plus particulièrement de 5 à 60 minutes et de préférence 5 à 40 minutes.

Concernant la température nécessaire au développement de la coloration, celle-ci est généralement comprise entre la température ambiante (15 à 25°C) et 220°C, plus spécialement entre la température ambiante et 80°C, et avantageusement entre 15 et 40°C.

Une fois la coloration souhaitée obtenue, après éventuellement un rinçage des fibres, on effectue habituellement un lavage des fibres avec un shampooing, on les rince et on les sèche ou on les laisse sécher.

Les exemples qui suivent illustrent la présente invention sans toutefois en limiter la portée.

### EXEMPLE 1

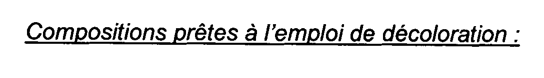

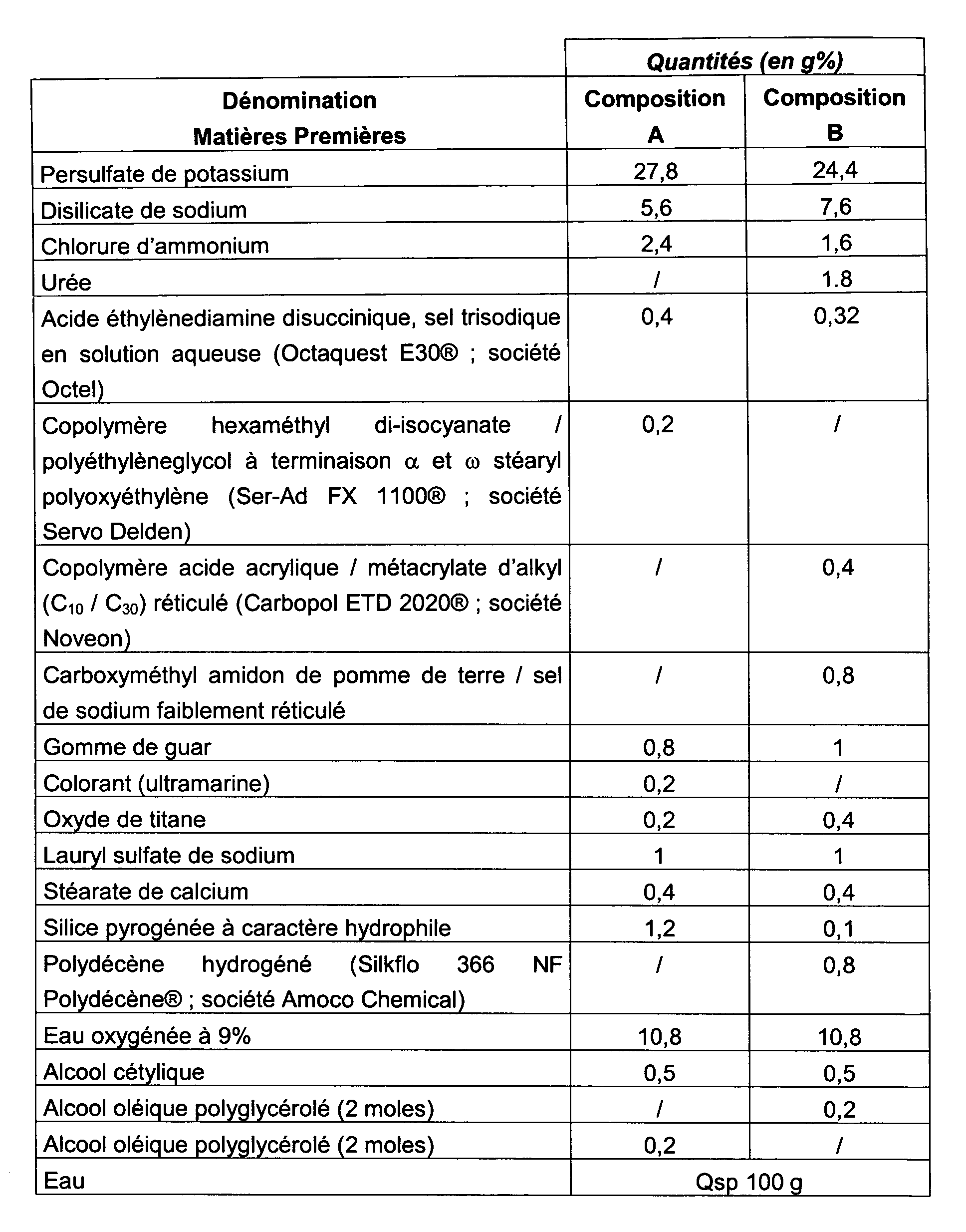

On applique la composition A pendant un temps de pose de 45 minutes, sous casque, sur des cheveux naturels foncés, puis on rince abondamment à l'eau.

On applique la composition B pendant un temps de pose de 30 minutes, sous casque, sur des cheveux naturels foncés, puis on rince abondamment à l'eau.

Dans chacun des deux cas, les compositions prêtes à l'emploi A et B présentent une texture idéale pour une application facile et rapide.

Elles permettent d'obtenir une décoloration puissante et homogène, avec des cheveux doux et brillants, et, faciles à démêler.

### EXEMPLE 2

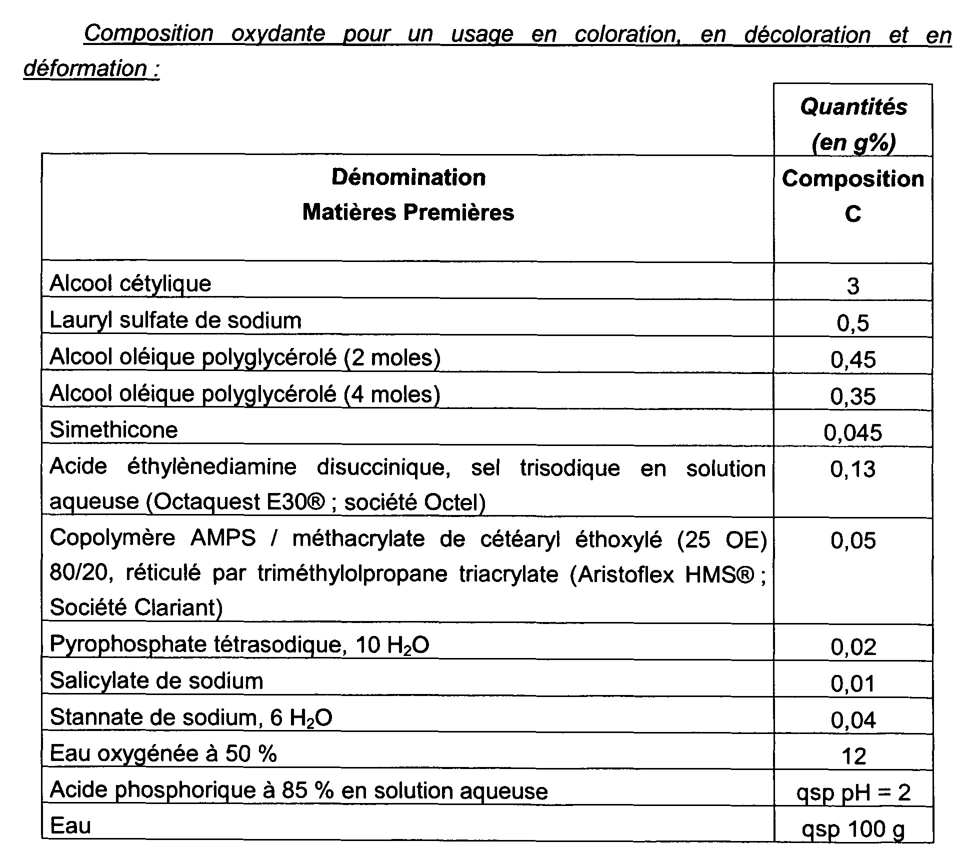

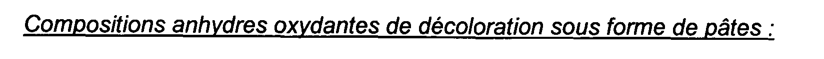

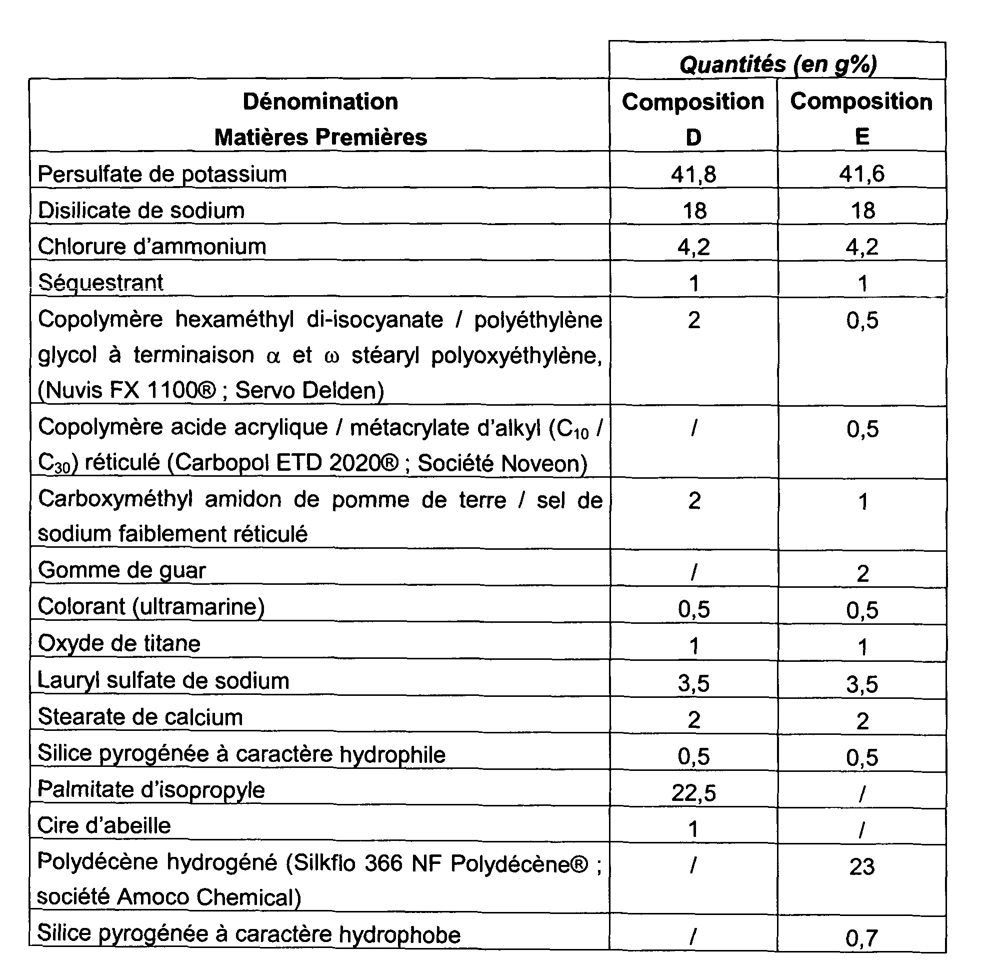

On mélange 40 g de la composition de décoloration D avec 80 g de la composition oxydante à base d'eau oxygénée C.

On applique le mélange décolorant prêt à l'emploi ainsi obtenu pendant un temps de pose de 45 minutes, sous casque, sur des cheveux naturels foncés, puis on rince abondamment à l'eau.

On mélange 40 g de la composition de décoloration E avec 60 g de la composition oxydante à base d'eau oxygénée C.

On applique le mélange décolorant prêt à l'emploi ainsi obtenu, pendant un temps de pose de 25 minutes, sous casque, sur des cheveux naturels foncés, puis on rince abondamment à l'eau.

Dans les deux cas, la composition prête à l'emploi ainsi obtenue présente une texture idéale pour une application facile et rapide et permet d'obtenir une décoloration puissante et homogène, avec des cheveux doux et brillants, et faciles à démêler.

### EXEMPLE 3

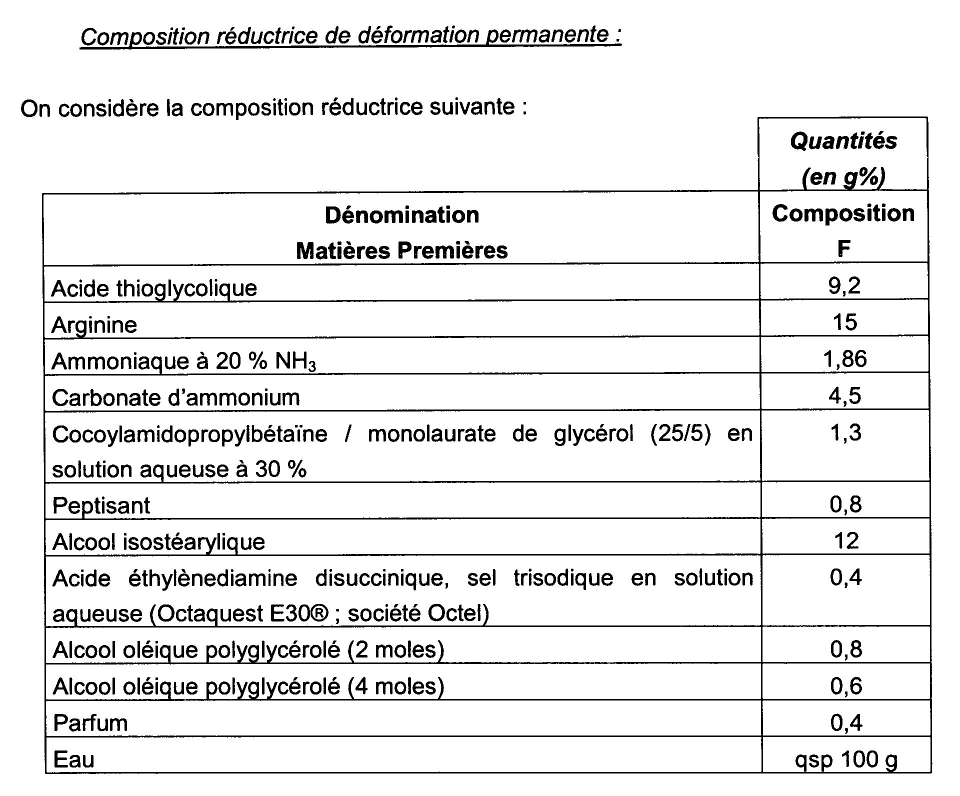

La composition réductrice F est appliquée sur une mèche de cheveux humides, préalablement enroulée sur un bigoudi de 9 mm de diamètre, pendant un temps de pose de 10 minutes.

Après quoi, on rince abondant à l'eau.

On applique ensuite la composition oxydante C pendant un temps de pose de10 minutes, puis on effectue un rinçage abondant à l'eau.

On déroule enfin les cheveux du bigoudi, puis on sèche.

On obtient une mèche ondulée.

### EXEMPLE 4

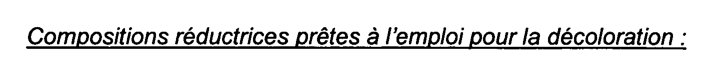

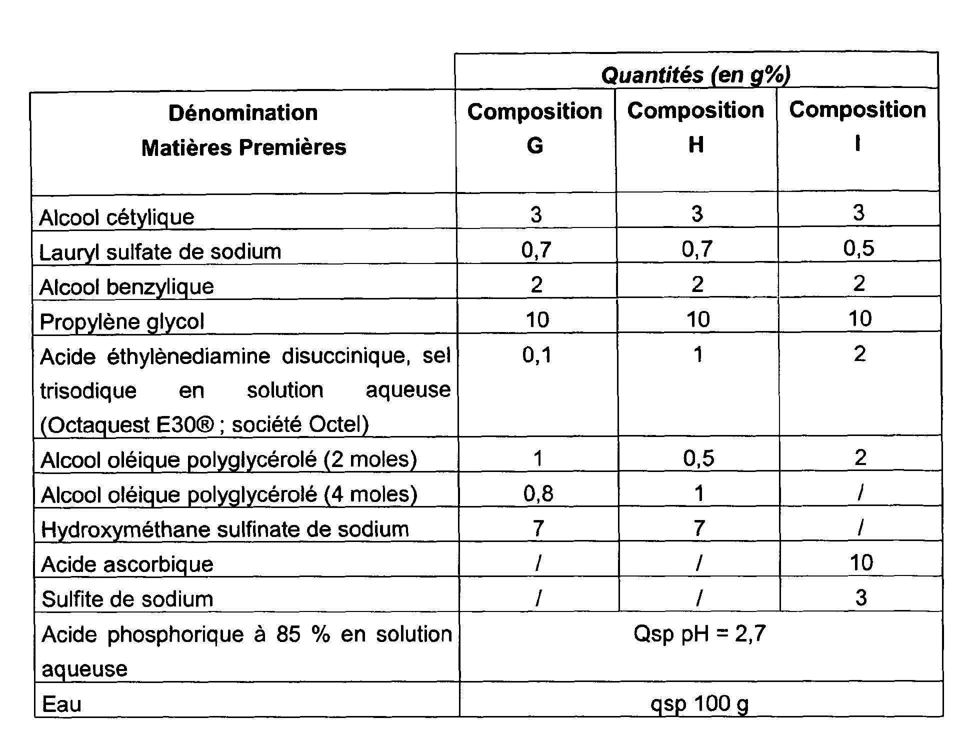

Les compositions de décoloration G, H et I sont appliquées sur des cheveux gris naturels à 90% de blancs, préalablement colorés par la nuance 6.66 de la gamme L'OREAL MAJIROUGE, avec un rapport de bain de 10 g de produit pour 1 g de cheveux.

Après 30 minutes de temps de pose, les mèches sont rincées, puis traitées 2 minutes à l'aide d'une solution aqueuse de peroxyde d'hydrogène à 3 %, puis rincées à nouveau.

On réalise ensuite un shampooing standard, puis on procède au séchage des cheveux.

On obtient une décoloration puissante et homogène.

Le reflet rouge intense a pratiquement disparu, laissant apparaître à nouveau les cheveux presque tels qu'ils étaient avant l'application de la teinture.

### EXEMPLE 5

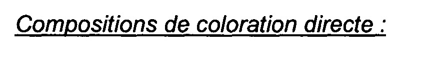

On a préparé les compositions suivantes (exprimées en grammes) :

| Composition oxydante : | |
|---|---|
| Alcool gras | 2,3 |
| Alcool gras oxyéthyléné | 0,6 |
| Amide gras | 0,9 |
| Glycérine | 0,5 |
| Peroxyde d'hydrogène | 7,5 |
| parfum | qs |
| Eau déminéralisée qsp | 100 |

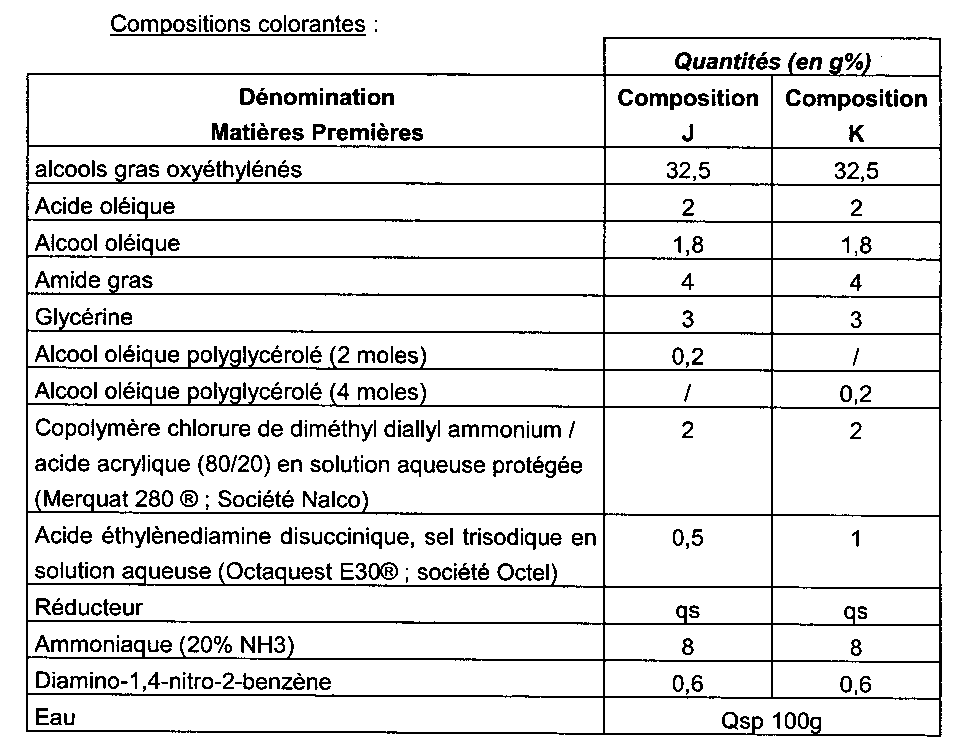

Les compositions colorantes ont été mélangées, au moment de l'emploi, dans un bol en plastique et pendant 2 minutes, à la composition oxydante donnée ci-dessus, à raison de 1 partie de composition colorante pour 1,5 parties de composition oxydante.

On a appliqué les mélanges obtenus sur des mèches de cheveux naturels à 90% de blancs et on a laissé poser 30 minutes.

Les compositions prêtes à l'emploi ainsi obtenues présentent une texture idéale pour une application facile (localisation facile) et rapide.

On a ensuite rincé les mèches à l'eau, on les a lavées au shampooing standard, à nouveau rincées à l'eau, puis séchées et démêlées.

Les cheveux ont été teints dans les deux cas dans une nuance rouge puissante, et les cheveux ne sont pas rêches.

### EXEMPLE 6

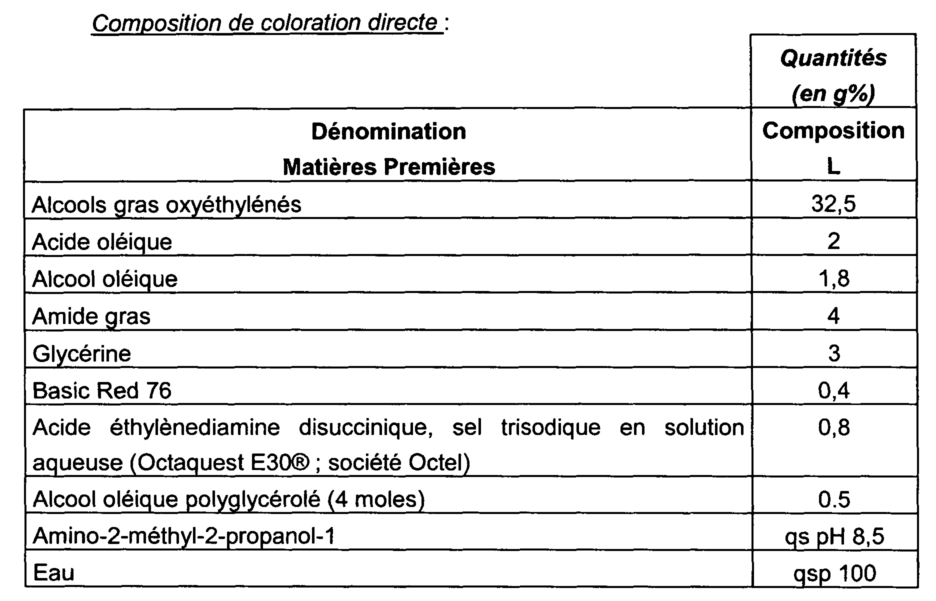

Cette composition a été appliquée trente minutes sur des cheveux gris à 90% de blancs.

Après rinçage et séchage les cheveux ont été colorés en rouge intense et les cheveux ne sont pas rêches.

### EXEMPLE 7

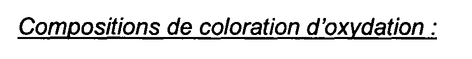

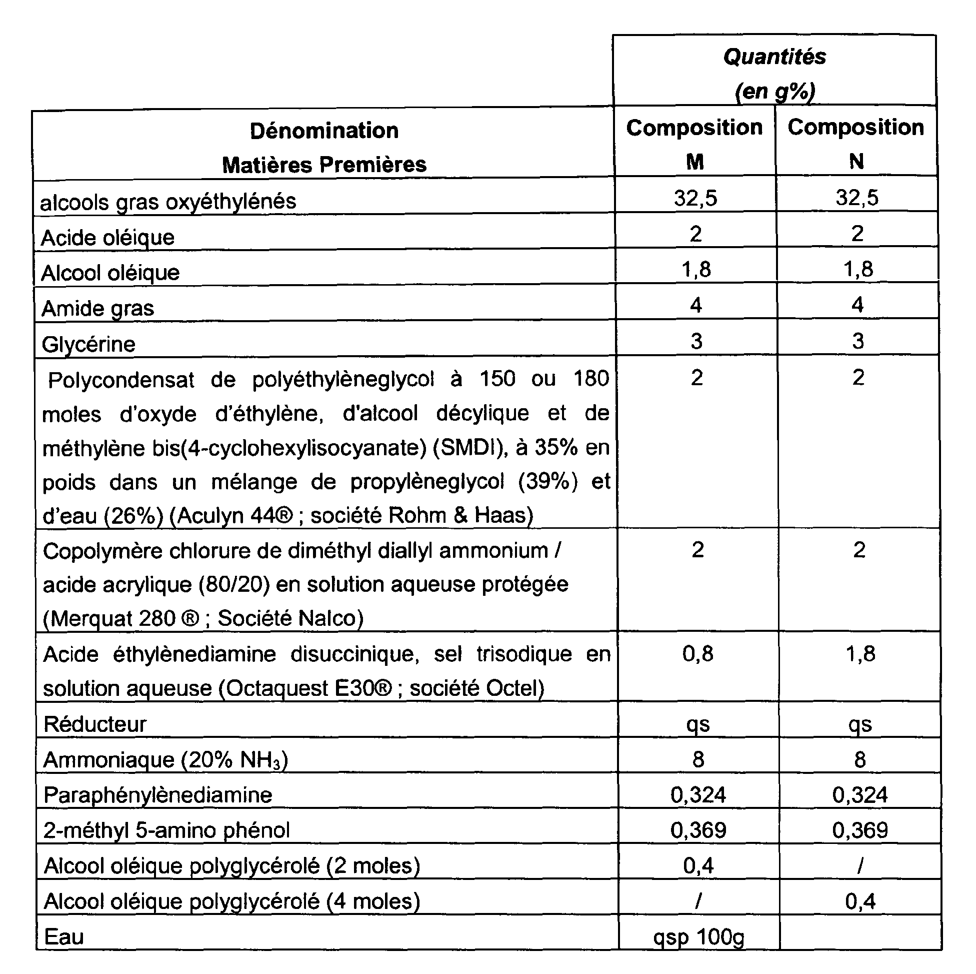

Les compositions colorantes ont été mélangées, au moment de l'emploi, dans un bol en plastique et pendant 2 minutes, à la composition oxydante C, à raison de 1 partie de composition colorante pour 1,5 parties de composition oxydante.

On a appliqué les mélanges obtenus sur des mèches de cheveux naturels à 90% de blancs et on a laissé poser 30 minutes.

Les compositions prêtes à l'emploi ainsi obtenues présentent une texture idéale pour une application facile (localisation facile) et rapide.

On a ensuite rincé les mèches à l'eau, on les a lavées au shampooing standard, à nouveau rincées à l'eau, puis séchées et démêlées.

Les cheveux ont été teints dans les deux cas dans une nuance pourpre-rouge puissante, et les cheveux ne sont pas rêches.

## Revendications

1. Composition destinée au traitement de fibres kératiniques humaines, comprenant :
- au moins un composé choisi parmi l'acide éthylènediamine-N,N'-disuccinique, un de ses sels, ou leur mélange,
- au moins un tensioactif glycérolé.

2. Composition selon la revendication précédente, **caractérisée en ce que** la teneur en composé est comprise entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

3. Composition selon l'une quelconque des revendications précédentes, **caractérisée en ce que** le tensioactif glycérolé est un alcool gras mono- ou poly- glycérolé de correspondant à la formule suivante : 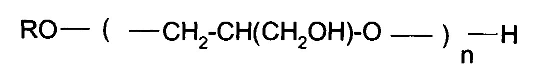 dans laquelle :
R représente un radical saturé ou insaturé, linéaire ou ramifié, comportant de 8 à 40 atomes de carbone et de préférence de 10 à 30 atomes de carbone ;
n représente un nombre compris entre 1 et 30 et de préférence entre 1 et 10.

4. Composition selon l'une quelconque des revendications précédentes, **caractérisée en ce que** le tensioactif glycérolé est choisi parmi l'alcool laurique à 4 moles de glycérol, l'alcool oléique à 4 moles de glycérol, l'alcool oléique à 2 moles de glycérol, l'alcool cétéarylique à 2 moles de glycérol, l'alcool cétéarylique à 6 moles de glycérol , l'alcool oléocétylique à 6 moles de glycérol, et l'octadécanol à 6 moles de glycérol, seuls ou en mélanges.

5. Composition selon l'une quelconque des revendications précédentes, **caractérisée en ce que** la teneur en tensioactif mono- ou poly-glycérolé représente de 0,01 à 30% en poids par rapport au poids de la composition, plus particulièrement de 0,05 à 20% en poids par rapport au poids de la composition et de préférence de 0,1 à 15% en poids par rapport au poids de la composition.

6. Composition selon l'une quelconque des revendications précédentes, **caractérisée en ce qu'**elle comprend au moins un agent oxydant.

7. Composition selon la revendication précédente, **caractérisée en ce que** l'agent oxydant est choisi parmi le peroxyde d'hydrogène, le peroxyde d'urée, les bromates ou ferricyanures de métaux alcalins, les persels tels que les perborates et les persulfates, seuls ou en mélanges.

8. Composition selon l'une quelconque des revendications 1 à 4, **caractérisée en ce qu'**elle comprend au moins un agent réducteur.

9. Composition selon la revendication précédente, **caractérisée en ce que** l'agent réducteur est choisi parmi les réductones, les composés présentant au moins une fonction thiol, (di)sulfure, sulfite, sulfiniques, ou leurs sels, ainsi que leurs mélanges.

10. Composition selon l'une quelconque des revendications précédentes, **caractérisée en ce qu'**elle comprend au moins un colorant direct, au moins une base d'oxydation éventuellement associée à au moins un coupleur, ou leurs mélanges.

11. Composition selon la revendication précédente, **caractérisée en ce que** le colorant direct est un colorant cationique ou non ionique, choisi parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

12. Composition selon la revendication 10, **caractérisée en ce que** la base d'oxydation est choisie parmi les o-phénylènediamines, les p-phénylènediamines, les bisphénylènediamines, les o-aminophénols, les p-aminophénols, les bis-p-aminophénols, les bases hétérocycliques, leurs sels d'addition avec un acide, ainsi que leurs mélanges.

13. Composition selon la revendication 10, **caractérisée en ce que** le coupleur est choisi parmi les m-aminophénols, les m-phénylènediamines, les m-diphénols, les naphtols, les coupleurs hétérocycliques, leurs sels d'addition avec un acide, ainsi que leurs mélanges.

14. Procédé de traitement des matières kératiniques humaines, consistant à appliquer sur lesdites matières, sèches ou humides, une composition selon l'une quelconque des revendications précédentes.

15. Procédé de déformation permanente des fibres kératiniques humaines consistant à mettre en contact une composition selon l'une quelconque des revendications 1 à 9, avec lesdites fibres pendant une durée suffisante pour obtenir l'effet souhaité.

16. Procédé de décoloration des fibres kératiniques humaines consistant à mettre en contact une composition selon l'une quelconque des revendications 1 à 9, avec lesdites fibres pendant une durée suffisante pour obtenir l'effet souhaité.

17. Procédé de coloration des fibres kératiniques humaines consistant à mettre en contact une composition selon l'une quelconque des revendications 1 à 7 et 10 à 13, avec lesdites fibres pendant une durée suffisante pour obtenir l'effet souhaité.
